# EUROPEAN PATENT SPECIFICATION

(11) **EP 1 939 622 B1**
(45) Date of publication and mention of the grant of the patent: **21.09.2011**
(21) Application number: 06810652.5
(22) Date of filing: 27.09.2006
(51) Int. Cl.: G01N 33/53, C12N 15/09, C12Q 1/68, G01N 21/64, G01N 33/543

(54) **METHOD OF FORMING AUTOAGGREGATE ON MICROPARTICLE AND METHOD OF DETECTING TARGET ANALYTE**
VERFAHREN ZUR BILDUNG VON AUTOAGGREGAT AUF EINEM MIKROPARTIKEL UND VERFAHREN ZUM NACHWEIS VON ZIELANALYTEN
PROCÉDÉ DE FORMATION D'UN AUTOAGRÉGAT SUR UNE MICROPARTICULE ET PROCÉDÉ DE DÉTECTION D'UN ANALYTE CIBLÉ

(30) Priority: 27.09.2005 JP 2005280863
(43) Date of publication of application: 02.07.2008
(73) Proprietor: Eisai R&D Management Co., Ltd., Tokyo 112-8088 (JP)
(72) Inventor: FUJIKAWA, Toshihiko, Kawasaki-shi 2120052 (JP); USUI, Mitsugu, Yokohama-shi, Kanagawa 2220032 (JP)
(74) Representative: Grünecker, Kinkeldey, Stockmair & Schwanhäusser Anwaltssozietät
(86) International application number: PCT/JP2006/319192
(87) International publication number: WO 2007/037282

(56) References cited:
- EP-A- 1 188 841
- WO-A1-02/18642
- WO-A1-03/029441
- WO-A1-2004/074480
- JP-A- 2002 501 184
- JP-A- 2002 506 984
- JP-A- 2003 088 390
- JP-A- 2005 091 014
- BRENNER SYDNEY ET AL: "Gene expression analysis by massively parallel signature sequencing (MPSS) on microbead arrays" NATURE BIOTECHNOLOGY, NATURE PUBLISHING GROUP, NEW YORK, NY, US, vol. 18, no. 6, 1 June 2000 (2000-06-01), pages 630-634, XP002199492 ISSN: 1087-0156
- DRESSMAN DEVIN ET AL: "Transforming single DNA molecules into fluorescent magnetic particles for detection and enumeration of genetic variations." PROCEEDINGS OF THE NATIONAL ACADEMY OF SCIENCES OF THE UNITED STATES OF AMERICA, vol. 100, no. 15, 22 July 2003 (2003-07-22), pages 8817-8822, XP002327377 ISSN: 0027-8424
- HAN MINGYONG ET AL: "Quantum-dot-tagged microbeads for multiplexed optical coding of biomolecules" NATURE BIOTECHNOLOGY, vol. 19, no. 7, July 2001 (2001-07), pages 631-635, XP002545495 ISSN: 1087-0156

### SEQUENCE LISTING

<110> Eisai R&D Management Co., Ltd.
<120> Method for forming self-assembly substance on micro particle and method for detecting target analyte
<130> 77161-P-PCT
<150> JP 2005-280863
   <151> 2005-09-27
<160> 34
<170> PatentIn version 3.1
<210> 1
   <211> 60
   <212> DNA
   <213> Artificial
<220>
   <223> Description of Artificial Sequence; Synthetic
<220>
   <221> misc_feature
   <222> (1)..(1)
   <223> Cy3 attached at the 5' end
<400> 1
   cgtatcaatg atagccgatc cgcctaagtt cgatatagtc cgcgtatact aagcgtaatg 60
<210> 2
   <211> 60
   <212> DNA
   <213> Artificial
<220>
   <223> Description of Artificial Sequence; Synthetic
<220>
   <221> misc_feature
   <222> (1)..(1)
   <223> Cy3 attached at the 5' end
<400> 2
   gatcggctat cattgatacg gactatatcg aacttaggcg cattacgctt agtatacgcg 60
<210> 3
   <211> 120
   <212> DNA
   <213> Artificial
<220>
   <223> Description of Artificial Sequence; Synthetic
<400> 3
   ggcggaggag acgcgggcac ggctgtccaa ggagctgcag gcggcgcagg cccggctggg 60
   cgcggacatg gaggacgtgt gcggccgcct ggtgcagtac cgcggcgagg tgcaggccat 120
<210> 4
   <211> 61
   <212> DNA
   <213> Artificial
<220>
   <223> Description of Artificial Sequence; Synthetic
<220>
   <221> misc_feature
   <222> (61)..(61)
   <223> amino group attached at the 3' end
<400> 4
   acacgtcctc catgtccgcg cccagccggg cctgcgccgc ctgcagctcc ttggacagcc 60
   g 61
<210> 5
   <211> 80
   <212> DNA
   <213> Artificial
<220>
   <223> Description of Artificial Sequence; Synthetic
<400> 5
   cgtatcaatg atagccgatc cgcctaagtt cgatatagtc cgcgtatact aagcgtaatg 60
   gtactgcacc aggcggccgc 80
<210> 6
   <211> 120
   <212> DNA
   <213> Artificial
<220>
   <223> Description of Artificial Sequence; Synthetic
<400> 6
   tgatgcccat gtcggtgcat gccttcacaa agcggaagaa tgtgtcagcc tcaaagaaaa 60
   gctgcgtgat gatgaaatcg gctcccgcag acaccttctc cttcaagtgc ttcaggtcag 120
<210> 7
   <211> 120
   <212> DNA
   <213> Artificial
<220>
   <223> Description of Artificial Sequence; Synthetic
<400> 7
   tgcctcagag caggaccttg gtctttcctt gtttgaagct ttgggctacg tggatgacca 60
   gctgttcgtg ttctatgatc atgagagtcg ccgtgtggag ccccgaactc catgggtttc 120
<210> 8
   <211> 120
   <212> DNA
   <213> Artificial
<220>
   <223> Description of Artificial Sequence; Synthetic
<400> 8
   tgaattagct gtatcgtcaa ggcactcttg cctacgccac cagctccaac taccacaagt     60
   ttatattcag tcattttcag caggcctctc tcccgcacct gggagccgct gagcctctgg    120
<210> 9
   <211> 61
   <212> DNA
   <213> Artificial
<220>
   <223> Description of Artificial Sequence; Synthetic
<220>
   <221> misc_feature
   <222> (61)..(61)
   <223> amino group attached at the 3' end
<400> 9
   ccgatttcat catcacgcag cttttctttg aggctgacac attcttccgc tttgtgaagg     60
   c                                                                     61
<210> 10
   <211> 61
   <212> DNA
   <213> Artificial
<220>
   <223> Description of Artificial Sequence; Synthetic
<220>
   <221> misc_feature
   <222> (61)..(61)
   <223> amino group attached at the 3' end
<400> 10
   gatcatagaa cacgaacagc tggtcatcca cgtagcccaa agcttcaaac aaggaaagac     60
   c                                                                     61
<210> 11
   <211> 61
   <212> DNA
   <213> Artificial
<220>
   <223> Description of Artificial Sequence; Synthetic
<220>
   <221> misc_feature
   <222> (1)..(1)
   <223> amino group attached at the 5' end
<400> 11
   aggtgcggga gagaggcctg ctgaaaatga ctgaatataa acttgtggta gttggagctg 60
   g 61
<210> 12
   <211> 80
   <212> DNA
   <213> Artificial
<220>
   <223> Description of Artificial Sequence; Synthetic
<400> 12
   cgtatcaatg atagccgatc cgcctaagtt cgatatagtc cgcgtatact aagcgtaatg 60
   ggagaaggtg tctgcgggag 80
<210> 13
   <211> 80
   <212> DNA
   <213> Artificial
<220>
   <223> Description of Artificial Sequence; Synthetic
<400> 13
   cgtatcaatg atagccgatc cgcctaagtt cgatatagtc cgcgtatact aagcgtaatg 60
   ctccacacgg cgactctcat 80
<210> 14
   <211> 80
   <212> DNA
   <213> Artificial
<220>
   <223> Description of Artificial Sequence; Synthetic
<400> 14
   tggcgtaggc aagagtgcct cgtatcaatg atagccgatc cgcctaagtt cgatatagtc 60
   cgcgtatact aagcgtaatg 80
<210> 15
   <211> 61
   <212> DNA
   <213> Artificial
<220>
   <223> Description of Artificial Sequence; Synthetic
<220>
   <221> misc_feature
   <222> (61)..(61)
   <223> amino group attached at the 3' end
<400> 15
   aagggtatgc ggaagcgagc accccagtct gagatggctc ctgccggtgt gagcctgagg 60
   g 61
<210> 16
   <211> 61
   <212> DNA
   <213> Artificial
<220>
   <223> Description of Artificial Sequence; Synthetic
<220>
   <221> misc_feature
   <222> (1)..(1)
   <223> amino group attached at the 5' end
<400> 16
   gggaatattc aggaactatt gctagtgagg ccaacactta cttgaattcc aagagcacac 60
   g 61
<210> 17
   <211> 61
   <212> DNA
   <213> Artificial
<220>
   <223> Description of Artificial Sequence; Synthetic
<220>
   <221> misc_feature
   <222> (1)..(1)
   <223> amino group attached at the 5' end
<400> 17
   gcctcccacc tgcgcaagct gcgtaagcgg ctcctccgcg atgccgatga cctgcagaag 60
   c 61
<210> 18
   <211> 61
   <212> DNA
   <213> Artificial
<220>
   <223> Description of Artificial Sequence; Synthetic
<220>
   <221> misc_feature
   <222> (61)..(61)
   <223> amino group attached at the 3' end
<400> 18
   tggtggtgca ggaggcattg ctgatgatct tgaggctgtt gtcatacttc tcatggttca 60
   c 61
<210> 19
   <211> 61
   <212> DNA
   <213> Artificial
<220>
   <223> Description of Artificial Sequence; Synthetic
<220>
   <221> misc_feature
   <222> (1)..(1)
   <223> amino group attached at the 5' end
<400> 19
   gacggtgagg gctcccagat cacaatgagg ggctgatcca ggcctgggtg ctccacctgg 60
   c 61
<210> 20
   <211> 61
   <212> DNA
   <213> Artificial
<220>
   <223> Description of Artificial Sequence; Synthetic
<220>
   <221> misc_feature
   <222> (61)..(61)
   <223> amino group attached at the 3' end
<400> 20
   tgacctgctg tgtcgagaat atccaagaga caggtttctc catcaattac tacttgcttc 60
   c 61
<210> 21
   <211> 61
   <212> DNA
   <213> Artificial
<220>
   <223> Description of Artificial Sequence; Synthetic
<220>
   <221> misc_feature
   <222> (61)..(61)
   <223> amino group attached at the 3' end
<400> 21
   aaagttgaac tagctagaat gagttaccag agagcagctt gggaggttga ttctggtcgt 60
   c 61
<210> 22
   <211> 61
   <212> DNA
   <213>' Artificial
<220>
   <223> Description of Artificial Sequence; Synthetic
<220>
   <221> misc_feature
   <222> (1)..(1)
   <223> amino group attached at the 5' end
<400> 22
   cctgaaaaca acgttctgtc ccccttgccg tcccaagcaa tggatgattt gatgctgtcc 60
   c 61
<210> 23
   <211> 61
   <212> DNA
   <213> Artificial
<220>
   <223> Description of Artificial Sequence; Synthetic
<220>
   <221> misc_feature
   <222> (61)..(61)
   <223> amino group attached at the 3' end
<400> 23
   cggggagcag cctctggcat tctgggagct tcatctggac ctgggtcttc agtgaaccat 60
   t 61
<210> 24
   <211> 61
   <212> DNA
   <213> Artificial
<220>
   <223> Description of Artificial Sequence; Synthetic
<220>
   <221> misc_feature
   <222> (1)..(1)
   <223> amino group attached at the 5' end
<400> 24
   ggaaccgctc attgccaatg gtgatgacct ggccgtcagg cagctcgtag ctcttctcca 60
   g 61
<210> 25
   <211> 80
   <212> DNA
   <213> Artificial
<220>
   <223> Description of Artificial Sequence; Synthetic
<400> 25
   cgtatcaatg atagccgatc cgcctaagtt cgatatagtc cgcgtatact aagcgtaatg 60
   tgttctgggt actacagcag 80
<210> 26
   <211> 80
   <212> DNA
   <213> Artificial
<220>
   <223> Description of Artificial Sequence; Synthetic
<400> 26
   gtcttcagtg aagctgcagg cgtatcaatg atagccgatc cgcctaagtt cgatatagtc 60
   cgcgtatact aagcgtaatg 80
<210> 27
   <211> 80
   <212> DNA
   <213> Artificial
<220>
   <223> Description of Artificial Sequence; Synthetic
<400> 27
   gcctggcagt gtaccaggcc cgtatcaatg atagccgatc cgcctaagtt cgatatagtc 60
   cgcgtatact aagcgtaatg 80

<210> 28
   <211> 80
   <212> DNA
   <213> Artificial
<220>
   <223> Description of Artificial Sequence; Synthetic
<400> 28
   cgtatcaatg atagccgatc cgcctaagtt cgatatagtc cgcgtatact aagcgtaatg 60
   ggccaggggt gctaagcagt 80
<210> 29
   <211> 80
   <212> DNA
   <213> Artificial
<220>
   <223> Description of Artificial Sequence; Synthetic
<400> 29
   acgtatatct ctgctcttcc cgtatcaatg atagccgatc cgcctaagtt cgatatagtc 60
   cgcgtatact aagcgtaatg 80
<210> 30
   <211> 80
   <212> DNA
   <213> Artificial
<220>
   <223> Description of Artificial Sequence; Synthetic
<400> 30
   cgtatcaatg atagccgatc cgcctaagtt cgatatagtc cgcgtatact aagcgtaatg 60
   tcattgcact gtactcctct 80
<210> 31
   <211> 80
   <212> DNA
   <213> Artificial
<220>
   <223> Description of Artificial Sequence; Synthetic
<400> 31
   cgtatcaatg atagccgatc cgcctaagtt cgatatagtc cgcgtatact aagcgtaatg 60
   tgctggctga aatggcaatg 80
<210> 32
   <211> 80
   <212> DNA
   <213> Artificial
<220>
   <223> Description of Artificial Sequence; Synthetic
<400> 32
   cggacgatat tgaacaatgg cgtatcaatg atagccgatc cgcctaagtt cgatatagtc 60
   cgcgtatact aagcgtaatg 80
<210> 33
   <211> 80
   <212> DNA
   <213> Artificial
<220>
   <223> Description of Artificial Sequence; Synthetic
<400> 33
   cgtatcaatg atagccgatc cgcctaagtt cgatatagtc cgcgtatact aagcgtaatg 60
   ctgctggtgc aggggccacg 80
<210> 34
   <211> 80
   <212> DNA
   <213> Artificial
<220>
   <223> Description of Artificial Sequence; Synthetic
<400> 34
   ggaggagctg gaagcagccg cgtatcaatg atagccgatc cgcctaagtt cgatatagtc     60
   cgcgtatact aagcgtaatg                                                 80

## Description

### Technical Field

The present invention relates to a signal amplification method using two kinds of oligonucleotides capable of forming a self assembly substance. More specifically, the present invention relates to a method of manufacturing a self assembly substance-bonded particle for detecting a target analyte, preferably plural target analytes at a high sensitivity on a fluorescent particle, and to a method of detecting a target analyte by flow cytometry.

### Background Art

In general, as a measurement method using microspheres, there are given latex agglutination, flow cytometry, and the like. However, the latex agglutination or the like can detect only one kind of target analyte at a time. However, some of methods using the flow cytometry can detect multiple items at the same time using plural kinds of microspheres with different fluorescence wavelengths and intensities (see Patent Document 1, for example). For example, in the case where a gene is detected by the method of detecting multiple items at the same time by the flow cytometry, gene amplification of the analyte with PCR or the like must be performed and the sensitivity of the detection is unsatisfactory. Meanwhile, in amplification by nucleic acid synthesis with PCR or the like, it is difficult to amplify multiple items at the same time. Therefore, a single amplification is not enough to amplify multiple items.

On the other hand, Usui et al. have reported a novel isothermal nucleic acid amplification method without using enzymes (Patent Documents 2 and 3). In this method, two kinds of oligonucleotides (referred to as probes) having base sequence regions that are complementary to each other are used to perform a self assembly reaction to form an assembly substance (polymer) of the probes. The quantification of the polymer can be applied to the detection of a target gene in a sample.
This method is a method of effectively detecting a test gene, where for example, a probe designed so as to have one complementary base sequence region including a base sequence complementary to that of a test gene in a sample is bonded to the test gene, and then a polymer of the probe is formed, and it is referred to as the PALSAR method.
Patent Document 1: JP 2002-501184 A
Patent Document 2: JP 3,267,576 B
Patent Document 3: WO 01/75157 A
EP-A-1 188 841 described self assembling oligonucleotides and analyte detection. The use of magnetic beads for separation purposes is described. However, fluorescently labeled beads are not disclosed. Further, this document is silent about the detection of the target analyte mediated by way of the beads.

### Disclosure of the Invention

### Problems to be solved by the Invention

In view of the current situations of the above-mentioned conventional technologies, the inventors of the present invention have made extensive studies to achieve the improvement of the sensitivity of detection by the PALSAR method and the simultaneous detection of plural items. An object of the present invention is to provide a method of detecting a target analyte on a microsphere with sensitivity improved and capable of detecting multiple items at the same time.

### Means for solving the Problems

The inventors of the present invention have completed the present invention for achieving highly sensitive simultaneous detection of multiple items by combining the simultaneous detection of multiple items by the flow cytometry and the PALSAR method.
A method of manufacturing a self assembly substance-bonded particle of the present invention includes: forming a self assembly substance having a regular double strand conformation on a microsphere having a fluorescent substance on a surface thereof (hereinafter, also referred to as fluorescent microsphere) by using two kinds of oligonucleotide probes having complementary base sequence regions which are hybridizable with each other; and manufacturing a microsphere by bonding the self assembly substance to the surface of the microsphere (hereinafter, also referred to as self assembly substance-bonded particle), wherein the two kinds of oligonucleotide probes are a pair of oligonucleotide probes including:
- a first probe that includes three or more of nucleic acid regions including at least a nucleic acid region X, a nucleic acid region Y, and a nucleic acid region Z in the stated order from a 5' end of the first probe; and
- a second probe that includes three or more of nucleic acid regions including at least a nucleic acid region X' complementary to the nucleic acid region X, a nucleic acid region Y' complementary to the nucleic acid region Y, and a nucleic acid region Z' complementary to the nucleic acid region Z in the stated order from a 5' end of the second probe.
The microsphere is preferably bonded to a target analyte to bond the microsphere to the self assembly substance via the target analyte.

### Examples of the target analyte include a nucleic acid.

The self assembly substance-bonded particle of the present invention is a microsphere having a fluorescent substance on the surface and bonded to a self assembly substance formed by using two kinds of oligonucleotide probes having complementary base sequence regions which are hybridizable with each other on the surface. The self assembly substance-bonded particle is produced by the above-mentioned method of manufacturing a self assembly substance-bonded particle of the present invention.

A method of detecting a target analyte of the present invention is a method of detecting the presence of at least one target analyte in a sample including the following steps (a) and (b):
(a) a first step of bonding a microsphere having a fluorescent substance on the surface and a target analyte to a self assembly substance formed by using two kinds of oligonucleotide probes having complementary base sequence regions which are hybridizable with each other via the target analyte to form a self assembly substance-bonded particle; and
(b) a second step of analyzing the self assembly substance-bonded particle to detect the target analyte, wherein the two kinds of oligonucleotide probes are a pair of oligonucleotide probes including:
   - a first probe that includes three or more of nucleic acid regions including at least a nucleic acid region X, a nucleic acid region Y, and a nucleic acid region Z in the stated order from a 5' end of the first probe; and
   - a second probe that includes three or more of nucleic acid regions including at least a nucleic acid region X' complementary to the nucleic acid region X, a nucleic acid region Y' complementary to the nucleic acid region Y, and a nucleic acid region Z' complementary to the nucleic acid region Z in the stated order from a 5' end of the second probe.

The microsphere preferably has a capture substance capable of specifically bonding to the target analyte.
The step (a) preferably includes: a step of using a probe capable of specifically bonding to the target analyte and having the same base sequence as a part or the whole of a base sequence of one oligonucleotide probe of the two kinds of oligonucleotide probes (hereinafter, referred to as assist probe) to form a complex including the assist probe and the target analyte that is specifically bonded to the capture substance on the microsphere; and a step of forming a self assembly substance bonded to the assist probe by using the plural kinds of oligonucleotide probes to form the self assembly substance-bonded particle.

In the step (b), the self assembly substance-bonded microsphere is analyzed preferably by flow cytometry.

In the present invention, it is preferable that at least one of the two kinds of oligonucleotide probes be labeled with a fluorescent substance or a substance capable of bonding to a fluorescent substance, and the fluorescent substance and the fluorescent substance on the surface of the microsphere have different fluorescence wavelengths or intensities.

Further, in the present invention, as the two kinds of oligonucleotide probes, preferably used is a pair of oligonucleotide probes including: a first probe that includes three or more of nucleic acid regions including at least a nucleic acid region X, a nucleic acid region Y, and a nucleic acid region Z in the stated order from a 5' end of the first probe; and a second probe that includes three or more of nucleic acid regions including at least a nucleic acid region X' complementary to the nucleic acid region X, a nucleic acid region Y' complementary to the nucleic acid region Y, and a nucleic acid region Z' complementary to the nucleic acid region Z in the stated order from a 5' end of the second probe.

A kit for detecting a target analyte of the present invention is a kit for detecting the presence of at least one target analyte in a sample including the following (a) and (b):
(a) a microsphere having a fluorescent substance on the surface; and
(b) a pair of oligonucleotide probes including: a first probe that includes three or more of nucleic acid regions including at least a nucleic acid region X, a nucleic acid region Y, and a nucleic acid region Z in the stated order from a 5' end of the first probe; and a second probe that includes three or more of nucleic acid regions including at least a nucleic acid region X' complementary to the nucleic acid region X, a nucleic acid region Y' complementary to the nucleic acid region Y, and a nucleic acid region Z' complementary to the nucleic acid region Z in the stated order from a 5' end of the second probe.

The microsphere preferably has a capture substance capable of specifically bonding to the target analyte.
A kit of the present invention preferably further includes an assist probe capable of specifically bonding to the target analyte and having the same base sequence as a part or the whole of a base sequence of one oligonucleotide probe of the pair of oligonucleotide probes.

### Effect of the Invention

According to the present invention, it is possible to improve the detection sensitivity for detecting a target analyte on a microsphere, and multiple items can be detected at the same time by a combination with the method described in Patent Document 1.

### Best Mode for carrying out the Invention

Hereinafter, the embodiments of the preset invention are described below with reference to the attached drawings. It should be understood that the embodiments described herein are merely exemplary and that many variations and modifications may be made without departing from the spirit and scope of the present invention.

The present invention is intended to form a self assembly substance (polymer) having a regular double strand conformation on a microsphere having a fluorescent substance on the surface by using two kinds of oligonucleotide probes having complementary base sequence regions which are hybridizable with each other to amplify signals, whereby a detection sensitivity in detection of a target analyte in a sample can be significantly improved.

A method of detecting a target analyte in a sample of the present invention will be described. First, a microsphere having a fluorescent substance on the surface is bonded to a target analyte, and a self assembly substance is formed on the microsphere via the target analyte by the PALSAR method, to thereby prepare a microsphere containing the self assembly substance (self assembly substance-bonded particle) (first step). Thereafter, the self assembly substance-bonded particle is analyzed to confirm the presence of the target analyte in the sample (second step).

### Examples of the target analyte include a nucleic acid.

Examples of a sample containing a target analyte to be used in the present invention include: organism-derived samples such as blood, serum, urine, feces, cerebrospinal fluid, tissue fluid, sputum, and cell cultures; and any samples potentially containing or potentially infected with viruses, bacteria, fungi, or the like. A target analyte in a sample may be extracted by a known method, and in some cases, the analyte may be amplified by a known method before use.

The microsphere is preferably one having a surface stained with at least one fluorescent dye or having a surface bonded to a stained nanoparticle.
In addition, the microsphere preferably includes polystyrene, polyacrylic acid, polyacrylonitrile, polyacrylamide, polyacrolein, polydimethylsiloxane, polybutadiene, polyisopyrene, polyurethane, polyvinylacetate, polyvinylchloride, polyvinylpyridine, polyvinylbenzylchloride, polyvinyltoluene, polyvinylidenechloride, polydivinylbenzene, polygycidyl methacrylate, polymethyl methacrylate, and a mixture thereof, a copolymer thereof, a synthetic compound thereof, a magnet, and a magnetic-responsive metal oxide.

Preferably, the microsphere contains a capture substance capable of specifically bonding to a target analyte, and the microsphere is bonded to the target analyte via a bond between the capture substance and target analyte. The capture substance is preferably an oligonucleotide having a base sequence complementary to that of one region in the target nucleic acid (hereinafter, referred to as capture probe). A method of bonding the capture substance to the microsphere is not particularly limited, and a microsphere immobilized with a capture substance by a known method may be used.

The two kinds of oligonucleotide probes to be used in the PALSAR method may be ones having complementary base sequence regions which are hybridizable with each other and capable of forming an assembly substance by self assembling, and the oligonucleotide probes described in Patent Documents 2 and 3 may be used.

The two kinds of oligonucleotide probes are preferably a pair of first and second oligonucleotide probes (also referred to as HCPs) including: a first probe that includes three nucleic acid regions including a nucleic acid region X, a nucleic acid region Y, and a nucleic acid region Z in the stated order from the 5' end and has a structure represented by the following chemical formula (1) (also referred to as HCP-1); and a second probe that includes three nucleic acid regions including a nucleic acid region X', a nucleic acid region Y', and a nucleic acid region Z' in the stated order from the 5' end and has a structure represented by the following chemical formula (2) (also referred to as HCP-2).

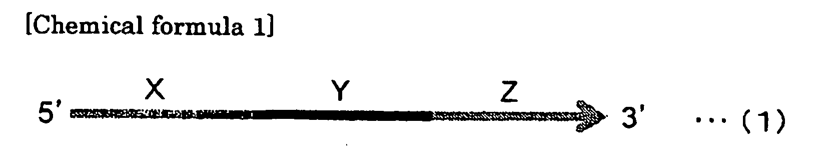

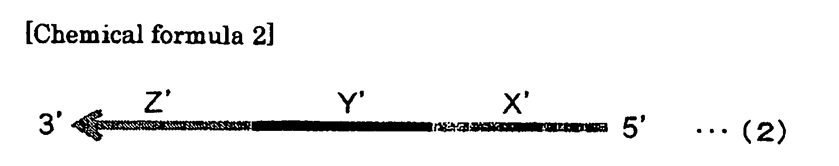

In the formulae (1) and (2), the nucleic acid regions X and X', Y and Y', and Z and Z' are complementary nucleic acid regions which are hybridizable with each other, and hybridization of the first probe and the second probe can form a polymer having a structure represented by the following formula (3).

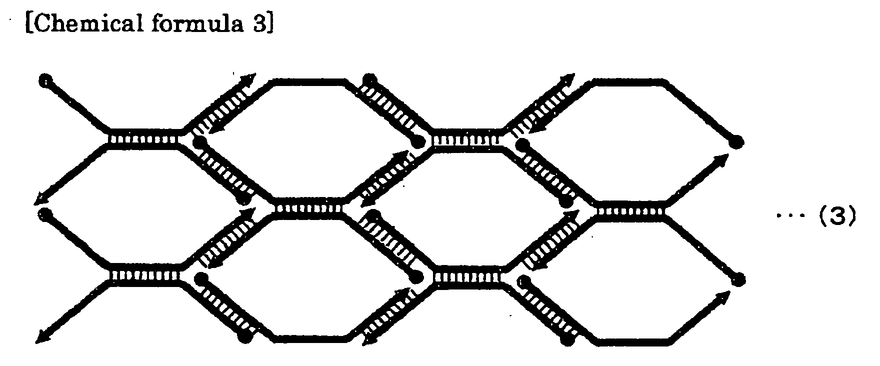

While a nucleic acid forming the pair of the oligonucleotides is usually DNA or RNA, a nucleic acid analogue may form the oligonucleotides. Examples of such a nucleic acid analogue include a peptide nucleic acid (PNA, see the pamphlet of International Patent Publication No. WO 92/20702, for example) and locked nucleic acid (LNA, see Koshkin AA et al., Tetrahedron 1998. 54, 3607-3630, Koshkin AA et al., J. Am. Chem. Soc., 1998. 120, 13252-13253 and Wahlestedt C et al., PNAS. 2000. 97, 5633-5638, for example). The pair of the oligonucleotide probes is generally composed of nucleic acids of the same kind, but may be composed of a pair of a DNA probe and an RNA probe. That is, the type of the nucleic acid of the probe may be selected from DNA, RNA or nucleic acid analogues (such as PNA and LNA). Also, it is not necessary that a probe is composed of a single type of nucleic acid, for example, DNA only, and, if necessary, for example, an oligonucleotide probe composed of DNA and RNA (a chimera probe) may be used in an aspect of the present invention.

A method of forming a self assembly substance on a microsphere preferably includes: bonding an assist probe having the same base sequence as a part or the whole of one oligonucleotide probe (for example, a first probe) of the two kinds of oligonucleotide probes to be used in the PALSAR method and capable of specifically bonding to a target analyte to the target analyte, to thereby form a complex including the assist probe, the target analyte, and a capture substance; and reacting the assist probe with the two kinds of oligonucleotide probes to bond the target analyte and the self assembly substance via the assist probe.
The order of the reaction between the capture substance and the target analyte and the reaction between the target analyte and the assist probe is not particularly limited, and one of the reactions may be firstly performed or the reactions may be performed at the same time.

The assist probe may be appropriately selected depending on the kind of target analyte. For example, in the case where the target analyte is a nucleic acid, an assist probe having a sequence complementary to that of one region in the target analyte (except for a region complementary to a capture probe) is preferably bonded to the target analyte by hybridization.

The two kinds of oligonucleotide probes to be used in the PALSAR method are preferably labeled with a labeling substance. For example, the oligonucleotide probes may be modified on the 5' end or the 3' end with a fluorescent substance such as Cy3 or a substance such as biotin or digoxigenin, and after the PALSAR reaction, biotin and digoxigenin may be bonded to a conjugate including anti-biotin or streptavidin and a fluorescent substance and a conjugate including anti-digoxigenin and a fluorescent substance, respectively, to indirectly bond the fluorescent substance.
The fluorescent substance is preferably selected so as to be different from a fluorescent substance bonded to the surface of a microsphere to distinguish between a fluorescence wavelength for recognizing a microsphere and a fluorescence wavelength for detecting a target analyte.

The two kinds of oligonucleotide probes to be used in the PULSAR method can self assemble by a hybridization reaction to form a self assembly substance. The number of the oligonucleotide probes used is not particularly limited, but it is within the range of 10² to 10¹⁵. The reaction conditions for formation of a self assembly substance are not particularly limited, and the reaction solution and reaction temperature may be selected depending on the kind of target analyte.

The reaction solution is preferably a buffer solution. The composition and concentration of a reaction buffer solution are not particularly limited, and a general buffer solution that is usually used for amplification of a nucleic acid is preferably used. In particular, in formation of a self assembly substance, a reagent capable of reducing a Tm value, such as tetraethylammonium chloride (TEAC) or tetramethylammonium chloride (TMAC) is preferably used (see William B. MELCHIOR et al. Proc. Nat. Acad. Sci. USA Vol. 70, No. 2, pp. 298-302, 1973, etc., for example). The pH is preferably within a usual range, and it is preferably pH 7.0 to 9.0.

The temperature condition for the formation of a self assembly substance is not particularly limited as long as probes to be used can hybridize with each other, and for example, in the case where a target analyte is a nucleic acid, the formation may be performed at a temperature within a usual range of 40 to 90°C, preferably within the range of 45 to 65°C.

As described above, a target analyte can be detected by: forming a self assembly substance-bonded particle, which is a microsphere including the target analyte and a self assembly substance; and analyzing the self assembly substance-bonded particle. A method of analyzing a self assembly substance-bonded particle is not particularly limited, and the self assembly substance-bonded particle is preferably analyzed by flow cytometry.

The detection method by flow cytometry requires one or more excitation lasers and two or more detection sensors with different wavelengths because it is necessary to detect a surface fluorescence for recognizing a fluorescent microsphere and a fluorescence caused by a fluorescent substance bonded to a self assembly substance in a self assembly substance-bonded particle. For example, in the case where fluorescent beads produced by Luminex Corporation are used as fluorescent microspheres, two kinds of fluorescence detection sensors are used for recognizing the fluorescent microspheres, and one detection sensor is used for detecting a target analyte. The apparatus to be used in the measurement is not particularly limited as long as it satisfies the above-mentioned conditions, and for example, Luminex 100 (manufactured by Luminex Corporation), FACS system (manufactured by Becton, Dickinson and Company), etc. may be used.

Hereinafter, for a case where a nucleic acid is detected as a target analyte (target substance), a method of detecting a target analyte of the present invention will be described in more detail.

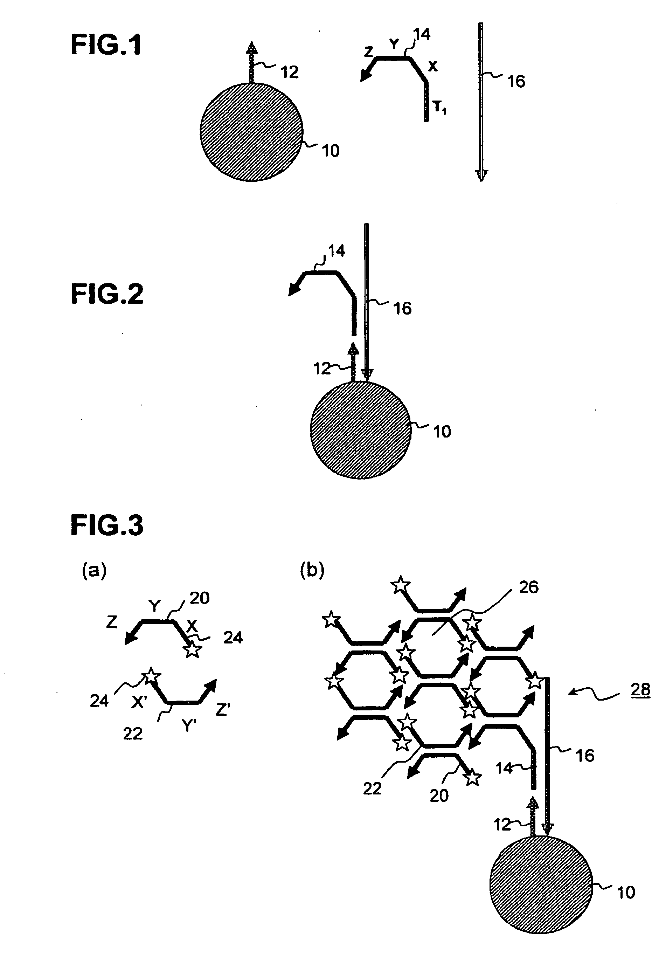

Figs. 1 to 3 are schematic diagrams showing in principle the first embodiment of the method of detecting a target analyte of the present invention. The first embodiment shows an example of a case where a nucleic acid is detected as a target substance. In Figs. 1 to 3, the reference number 10 designates a fluorescently-labeled microsphere, the reference number 16 designates a target nucleic acid, and the reference number 12 designates a capture probe having a base sequence complementary to that of the target nucleic acid. The reference numbers 20 and 22 designate two kinds of oligonucleotide probes to be used for formation of a self assembly substance, and the figure shows a case where a pair of HCPs [20: HCP-1 (nucleic acid region XYZ), 22: HCP-2 (nucleic acid region X'Y'Z')] are used. Both the probes are previously labeled with a fluorescent substance 24. The reference number 14 designates an assist probe having the same nucleic acid region (XYZ) as that of HCP-1 and having a nucleic acid region (T₁) complementary to that of the target nucleic acid 20.

As shown in Fig. 1, in the case where the microsphere 10 immobilized with the capture probe 12, the assist probe 14, and the target nucleic acid 16 have mutually complementary regions as shown in Fig. 2, hybridization is performed at the two sites to form a complex. Thereafter, as shown in Fig. 3, on the complex, a pair of HCPs previously labeled with the fluorescent substance 24 [HCP-1 (the reference number 20) and HCP-2 (the reference number 22)] are used to form the self assembly substance 26 through a self assembly reaction. Then, an analysis of the resultant self assembly substance-bonded particle 28, for example, signal measurement by flow cytometry is performed to confirm the presence or absence of a target substance.

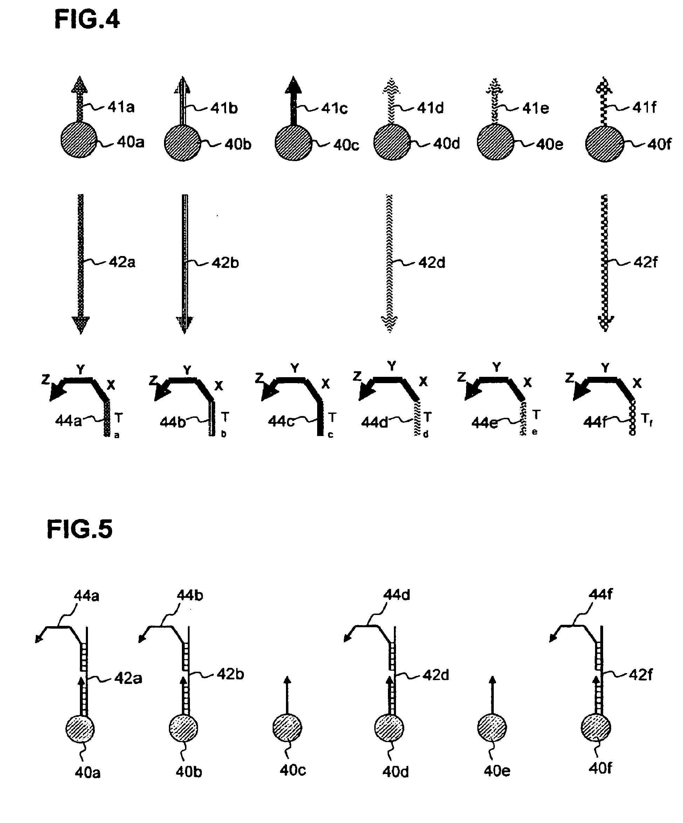

Figs. 4 to 6 are schematic diagrams showing in principle the second embodiment of the method of detecting a target analyte of the present invention. The second embodiment shows an example of a case where six kinds of genes are detected at the same time as target substances. Figs. 4 to 6 show examples of cases where four kinds of genes (42a, 42b, 42d, and 42f) of six kinds of genes (42a to 42f) serving as target substances are present in a sample.
In Figs. 4 to 6, the reference numbers 40a to 40f designate microspheres, which are bonded to capture probes 41a to 41f corresponding to six kinds of target genes 42a to 42f, respectively, and the surfaces of the microspheres have different fluorescent substances. The reference numbers 44a to 44f designate assist probes, which have nucleic acid regions (Tₐ to T_{f}) complementary to those of the six kinds of target genes 42a to 42f, respectively, and each have the same nucleic acid region (XYZ) as that of HCP-1.

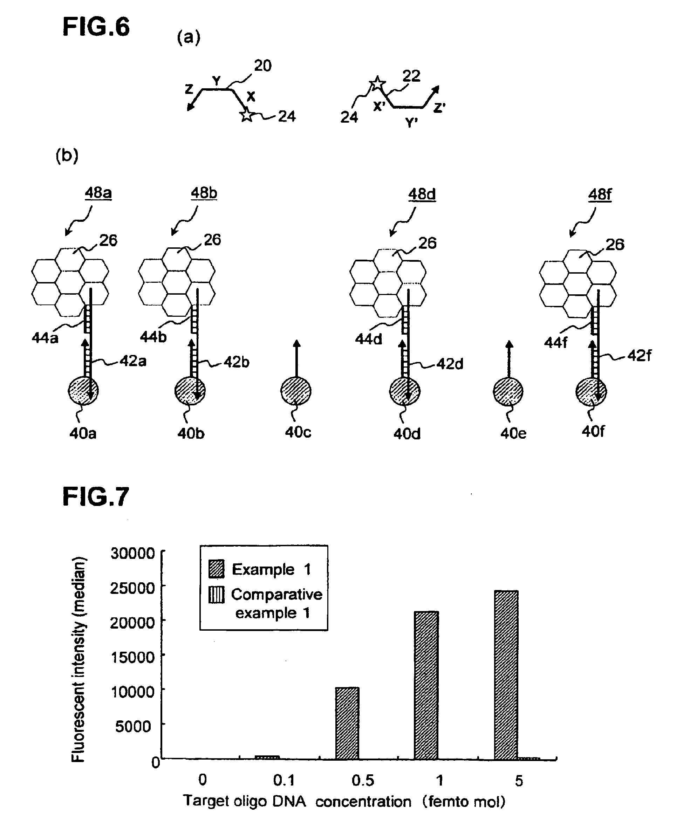

As shown in Fig. 4, if a sample containing the fluorescent microspheres 40a to 40f immobilized with the capture probes 41a to 41f corresponding to six kinds of genes, respectively, the assist probes 44a to 44f corresponding to the six kinds of genes, and the target nucleic acids corresponding to four kinds of genes of the six kinds of the genes is added to the same reaction solution to perform a hybridization reaction, complexes are formed only on the four kinds of microspheres of which target nucleic acids are present as shown in Fig. 5. Thereafter, as shown in Fig. 6, regardless of the kinds of the target nucleic acids, addition of only a pair of HCPs (22 and 24) forms the self assembly substance 26 on the complexes by a self assembly reaction, to thereby yield the self assembly substance-bonded particles (48a, 48b, 48d, and 48f).

If the reaction solution after formation of the self assembly substances is subjected to measurement using a flow cytometer, the six kinds of target substances can be distinguished based on the fluorescence wavelengths or intensities on the surfaces of the microspheres. Moreover, the fluorescences attributed to the self assembly substances are measured, and thereby plural kinds of target substances can be detected at the same time at a high sensitivity.

### Examples

The present invention is more specifically described by means of the examples below, but it will be understood that the examples are presented by way of illustration only and should not be construed as any limitation on the present invention.

### (Example 1 and Comparative Example 1)

The availability of the PALSAR method on a microsphere was demonstrated. Fluorescent intensities of positive signals for different concentrations (0 to 5 fmol) of a target gene (hereinafter, referred to as target oligo DNA) were compared using one kind of microspheres based on the presence or absence of the PALSAR method.

### 1. Materials

Oligonucleotide probes having the following base sequences (HCP-1A and HCP-2A) were used as a pair of HCPs to be used in the PALSAR method.
Base sequence of HCP-1A (SEQ ID NO. 1) Base sequence of HCP-2A (SEQ ID NO. 2)

A target oligo DNA used was target oligo DNA-1 derived from Apolipoprotein E (ApoE) and having the following base sequence. Base sequence of target oligo DNA-1 (SEQ ID: 3)

An capture probe used was the following capture probe-1 having a base sequence complementary to that of the target oligo DNA-1. Base sequence of capture probe-1 (SEQ ID NO. 4)

An assist probe used was the following assist probe-1 having the same base sequence as that of the HCP-LA and a base sequence complementary to that of the target oligo DNA-1.
Base sequence of assist probe-1 (SEQ ID NO. 5)

Microspheres used were carboxyl group-coated beads (manufactured by Luminex Corporation). The above-mentioned capture probe-1 was immobilized on the microspheres with EDC reagent and used in Example 1.

### 2. Method

### (2-1) First hybridization

A first hybridization solution having the following composition (total volume: 50 µL) was prepared, and the solution was allowed to react at 95°C for 2 minutes, followed by reacting at 68°C for 120 minutes, and maintained at 15°C.

### (Composition of the first hybridization solution)

Microspheres (immobilized with the capture probe): 500 particles
Assist probe-1: 1.5 pmol
Target oligo DNA-1: 0, 0.1, 0.5, 1, or 5 fmol
1.5M TMAC
0.1% N-Lauroylsarcosine
50 mM Tris-HCl (pH 8.0)
4 mM EDTA (pH 8.0)

### (2-2) Second hybridization (PALSAR method)

A second hybridization solution having the following composition (total volume: 50 µL) was prepared, heat-treated at 95°C for 2 minutes, and added to the solution obtained after the first hybridization reaction (final volume: 100 µL), and the resultant solution was allowed to react at 68°C for 60 minutes and maintained at 15°C.
(Composition of second hybridization solution
HCP-1A: 50 pmol (Example 1) or 0 (Comparative Example 1)
HCP-2A: 50 pmol
1.5 M TMAC
0.1% N-Lauroylsarcosine
50 mM Tris-HCl (pH 8.0)
4 mM EDTA (pH 8.0)

### (2-3) Measurement

After the second hybridization reaction, measurement was performed using a flow cytometer. The measurement was performed using Luminex 100 (manufactured by Luminex Corporation) as a flow cytometer to measure fluorescent intensities of about 100 microspheres for each item, and a median was calculated. The results are shown in Table 1 and Fig. 7. The numerical values are ones calculated by subtracting a blank value.

**[Table 1]**

| Target oligo DNA (femto mol) | Example 1 | Comparative Example 1 |
|---|---|---|
| 0 | 0 | 0 |
| 0.1 | 452 | 11 |
| 0.5 | 10,316 | 21 |
| 1 | 21,198 | 44 |
| 5 | 24,168 | 251 |

As shown in Table 1 and Fig. 7, the median in the case of 0.1 fmol of Example 1 is higher than that in the case of 5 fmol of Comparative Example 1 performed without the PALSAR method, which revealed that the sensitivity was significantly improved by signal amplification through the PALSAR method.

### (Examples 2-1 to 2-3)

Four kinds of fluorescent microspheres were detected at the same time by the PALSAR method. Four different kinds of capture probes were bonded to four kinds of microspheres with different fluorescent intensities, respectively, and one kind (Example 2-1), two kinds (Example 2-2), or four kinds (Example 2-3) of target oligo DNAs corresponding to the probes were mixed to amplify signals at the same time by the PALSAR method, followed by measurement using a flow cytometer.

### 1. Materials

HCP-1A and HCP-2A, which were used in Example 1, were used as a pair of HCPs to be used in the PALSAR method.

Target oligo DNAs used were target oligo DNA-1 used in Example 1, target oligo DNA-2 derived from 5,10-Methylenetetrahydrofolate Reductase (MTHFR) and having the following base sequence, target oligo DNA-3 derived from hemochromatosis gene (HFE) and having the following base sequence, and target oligo DNA-4 derived from KRAS and having the following base sequence.
Base sequence of target oligo DNA-2 (SEQ ID NO. 6) Base sequence of target oligo DNA-3 (SEQ ID NO. 7) Base sequence of target oligo DNA-4 (SEQ ID NO. 8)

Capture probes used were capture probes-1 to 4 having base sequences complementary to those of the target oligo DNAs-1 to 4, respectively. The capture probe-1 is one used in Example 1.
Base sequence of capture probe-2 (SEQ ID NO. 9) Base sequence of capture probe-3 (SEQ ID NO. 10) Base sequence of capture probe-4 (SEQ ID NO. 11)

Assist probes used were assist probes-1 to 4 having base sequences complementary to those of the target oligo DNAs-1 to 4, respectively, and each have the same base sequence as that of the HCP-1A. The assist probe-1 is one used in Example 1.
Base sequence of assist probe-2 (SEQ ID NO. 12) Base sequence of assist probe-3 (SEQ ID NO. 13) Base sequence of assist probe-4 (SEQ ID NO. 14)

Microspheres used were four kinds of carboxyl group-coated beads (manufactured by Luminex Corporation). Any of the above-mentioned capture probes-1 to 4 was immobilized on the microspheres.

### 2. Method

Reactions and measurement were performed in the same way as Example 1 except that the microspheres, target oligo DNAs, and assist probes in the first hybridization solution were changed as follows.
(a) Microsphere: 500 particles (immobilized with the capture probes) of each of the four kinds of microspheres
(b) Target oligo DNA: 0, 0.1, 0.5, 1, or 5 fmol of target oligo DNA-1 (Example 2-1), target oligo DNAs-1 and 2 (Example 2-2), or target oligo DNAs-1 to 4 (Example 2-3)
c) Assist probe: 0.375 pmol of each of assist probes-1 to 4 (total of the mixed four kinds of the probes: 1.5 pmol)

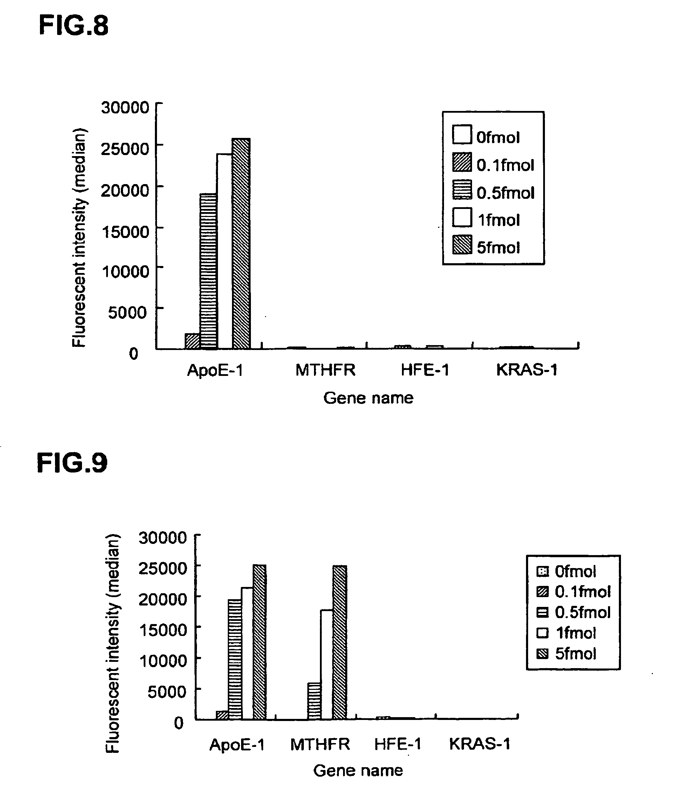

The results of Examples 2-1 to 2-3 are shown in Fig. 8 to Fig. 10, respectively. The result of the fluorescent intensity of the microsphere bonded to capture probe-1 is shown as ApoE-1, the result of the fluorescent intensity of the microsphere bonded to capture probe-2 is shown as MTHFR, the result of the fluorescent intensity of the microsphere bonded to capture probe-3 is shown as HFE-1, and the result of the fluorescent intensity of the microsphere bonded to capture probe-4 is shown as KRAS-1. The values are ones calculated by subtracting a blank value.

As shown in Figs. 8 to 10, the signals of ApoE-1 were found to be high and almost the same as in the case of using one kind of target oligo DNA and in the cases of using plural kinds (two and four kinds) of targets, which revealed that signal amplification by the PALSAR method was not affected by the number of items. Meanwhile, extremely high signals were detected only for the targets added, and therefore the specificity of the PALSAR method was found to be extremely high.

### (Example 3)

14 kinds of fluorescent microspheres were detected at the same time by the PALSAR method. Different kinds of capture probes were separately bonded to 14 kinds of microspheres with different fluorescent intensities, and four kinds of target oligo DNAs corresponding to the probes were mixed to amplify signals at the same time by the PALSAR method, followed by measurement using a flow cytometer.

Reactions and measurement were performed in the same way as Example 2 except that the microspheres, assist probes, and target oligo DNAs in the first hybridization solution were changed as follows.
(a) Microsphere: 500 beads of each of 14 kinds of carboxyl group-coated beads (manufactured by Luminex Corporation) immobilized with 14 kinds of capture probes (capture probes-1 to 14)
(b) Target oligo DNA: 0.5 fmol of each of target oligo DNAs-1 to 4
c) Assist probe: 0.11 pmol of each of assist probes-1 to 14 (total of the mixed 14 kinds of probes: 1.5 pmol)

The HCPs-1A and 2A, target oligo DNAs 1 to 4, capture probes-1 to 4, and assist probes-1 to 4 are ones used in Example 2, and capture probes-5 to 14 and assist probes-5 to 14 were designed so as to correspond to the following genes.
Capture probe-5 and assist probe-5: Angiotensinogen (Ang)
Capture probe-6 and assist probe-6: ApolipoproteinB100 (ApoB)
Capture probe-7 and assist probe-7: ApoE (a region different from target oligo DNA-1 and referred to as ApoE-2)
Capture probe-8 and assist probe-8: Glyceraldehyde-3-PhosphateDehydrogenase (G3P)
Capture probe-9 and assist probe-9: HFE (a region different from target oligo DNA-3 and referred to as HFE-2)
Capture probe-10 and assist probe-10: KRAS (a region different from target oligo DNA-4 and referred to as KRAS-2)
Capture probe-11 and assist probe-11: MediumChainAcyl-CoADehydrogenase (MCAD)
Capture probe-12, 13 and assist probe-12, 13: one of region in p53 (referred to as p53-1 and p53-2, respectively)
Capture probe-13 and assist probe-13: B-actin (BA)

Base sequence of capture probe-5 (SEQ ID NO. 15) Base sequence of capture probe-6 (SEQ ID NO. 16) Base sequence of capture probe-7 (SEQ ID NO. 17) Base sequence of capture probe-8 (SEQ ID NO. 18) Base sequence of capture probe-9 (SEQ ID NO. 19) Base sequence of capture probe-10 (SEQ ID NO. 20) Base sequence of capture probe-11 (SEQ ID NO. 21) Base sequence of capture probe-12 (SEQ ID NO. 22) Base sequence of capture probe-13 (SEQ ID NO. 23) Base sequence of capture probe-14 (SEQ ID NO. 24)

Base sequence of assist probe-5 (SEQ ID NO. 25) Base sequence of assist probe-6 (SEQ ID NO. 26) Base sequence of assist probe-7 (SEQ ID NO. 27) Base sequence of assist probe-8 (SEQ ID NO. 28)

Base sequence of assist probe-9 (SEQ ID NO. 29) Base sequence of assist probe-10 (SEQ ID NO. 30) Base sequence of assist probe-11 (SEQ ID NO. 31) Base sequence of assist probe-12 (SEQ ID NO. 32) Base sequence of assist probe-13 (SEQ ID NO. 33) Base sequence of assist probe-14 (SEQ ID NO. 34)

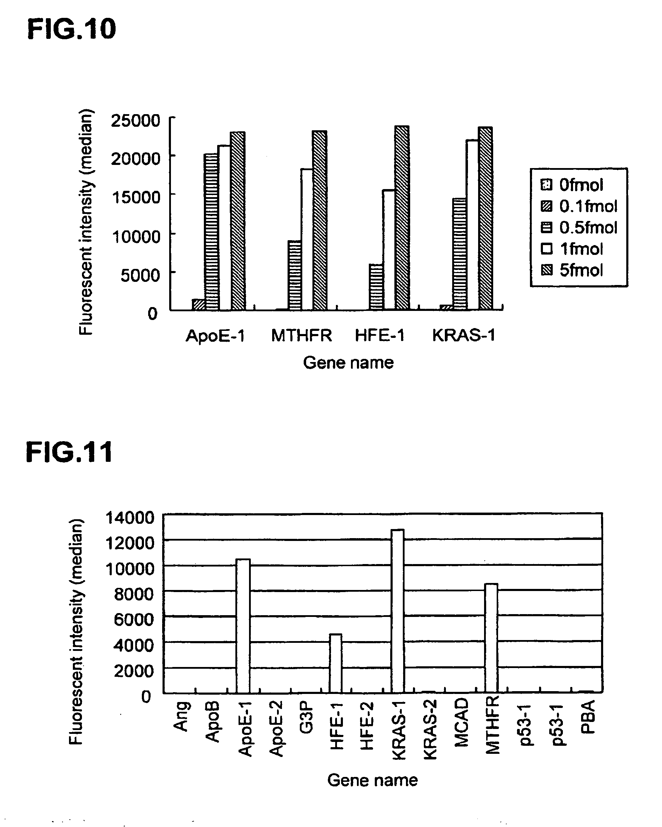

The results are shown in Table 2 and Fig. 11. The values are ones calculated by subtracting a blank value, and the values calculated to be below 0 are shown as 0. As shown in Table 2 and Fig. 11, the different 14 kinds of microspheres were found to react with only the targets added and provide high signals.

**[Table 2]**

| Gene name | Ang | ApoB | ApoE-1 | ApoE-2 | G3P | HFE-1 | HFE-2 |
|---|---|---|---|---|---|---|---|
| Fluorescent intensity | 0 | 0 | 10472.5 | 12 | 0 | 4,621 | 0 |

| Gene name | KRAS-1 | KRAS-2 | MCAD | MTHFR | p53-1 | p53-2 | BA |
|---|---|---|---|---|---|---|---|
| Fluorescent intensity | 12,709 | 50 | 0 | 8,505 | 34 | 0 | 76.5 |

### Brief Description of the Drawings

Fig. 1 is a schematic illustration showing the first embodiment of the method of detecting a target analyte of the present invention, which shows a microsphere bonded to a capture probe, an assist probe, and a target nucleic acid.
Fig. 2 is a schematic illustration showing the first embodiment of the method of detecting a target analyte of the present invention, which shows formation of a complex by hybridization of the capture probe, the target nucleic acid, and the assist probe on the microsphereshown in Fig. 1.
Fig. 3 is a schematic illustration showing the first embodiment of the method of detecting a target analyte of the present invention, where (a) shows a pair of oligonucleotide probes to be used in the PALSAR method, and (b) shows formation of a self assembly substance on the complex shown in Fig. 2.
Fig. 4 is a schematic illustration showing the second embodiment of the method of detecting a target analyte of the present invention, which shows microspheres bonded to capture probes, assist probes, and target nucleic acids.
Fig. 5 is a schematic illustration showing the second embodiment of the method of detecting a target analyte of the present invention, which shows formation of complexes only on four kinds of particles of which target nucleic acids are present.
Fig. 6 is a schematic illustration showing the second embodiment of the method of detecting a target analyte of the present invention, which shows formation of self assembly substances on the complexes shown in Fig. 5.
Fig. 7 is a graph showing the results of Examples 1 and Comparative Example 1.
Fig. 8 is a graph showing the results of Example 2-1.
Fig. 9 is a graph showing the results of Example 2-2.
Fig. 10 is a graph showing the results of Example 2-3.
Fig. 11 is a graph showing the results of Example 3.

### Description of Reference Numerals

10, 40a∼40f microspheres, 12, 41a∼41f: capture probes, 14, 44a∼44f: assist probes, 16, 42a, 42b, 42d, 42f: target nucleic acids, 20: HCP-1, 22: HCP-2, 24: fluorescent substances 26: self assembly substance, 28, 48a, 48b, 48d, 48f: self assembly substance-bonded particles.

## Claims

1. A method of manufacturing a self assembly substance-bonded particle comprising:
forming a self assembly substance having a regular double strand conformation on a microsphere having a fluorescent substance on a surface thereof by using two kinds of oligonucleotide probes having complementary base sequence regions which are hybridizable with each other; and
bonding the self assembly substance to the surface of the microsphere to manufacture a self assembly substance-bonded particle,
wherein the two kinds of oligonucleotide probes are a pair of oligonucleotide probes including:
a first probe that includes three or more of nucleic acid regions including at least a nucleic acid region X, a nucleic acid region Y, and a nucleic acid region Z in the stated order from a 5' end of the first probe; and
a second probe that includes three or more of nucleic acid regions including at least a nucleic acid region X' complementary to the nucleic acid region X, a nucleic acid region Y' complementary to the nucleic acid region Y, and a nucleic acid region Z' complementary to the nucleic acid region Z in the stated order from a 5' end of the second probe.

2. The method according to Claim 1, wherein at least one of the two kinds of oligonucleotide probes is labeled with a fluorescent substance or a substance capable of bonding to a fluorescent substance, and the fluorescent substance and the fluorescent substance on the surface of the microsphere have different fluorescence wavelengths or intensities.

3. The method according to Claims 1 or 2, wherein the microsphere is bonded to a target analyte to bond the microsphere to the self assembly substance via the target analyte.

4. A self assembly substance-bonded particle produced by the method according to any one of Claims 1 to 3.

5. A method of detecting the presence of at least one target analyte in a sample, comprising the following steps (a) and (b):
(a) a first step of bonding a microsphere having a fluorescent substance on the surface and a target analyte to a self assembly substance formed by using two kinds of oligonucleotide probes having complementary base sequence regions which are hybridizable with each other via the target analyte to form a self assembly substance-bonded particle; and
(b) a second step of analyzing the self assembly substance-bonded particle to detect the target analyte,
wherein the two kinds of oligonucleotide probes are a pair of oligonucleotide probes including:
a first probe that includes three or more of nucleic acid regions including at least a nucleic acid region X, a nucleic acid region Y, and a nucleic acid region Z in the stated order from a 5' end of the first probe; and
a second probe that includes three or more of nucleic acid regions including at least a nucleic acid region X' complementary to the nucleic acid region X, a nucleic acid region Y' complementary to the nucleic acid region Y, and a nucleic acid region Z' complementary to the nucleic acid region Z in the stated order from a 5' end of the second probe.

6. The method according to Claim 5, wherein the microsphere has a capture substance capable of specifically bonding to the target analyte.

7. The method according to Claim 6, wherein the step (a) includes:
a step of using an assist probe capable of specifically bonding to the target analyte and having the same base sequence as a part or the whole of a base sequence of one oligonucleotide probe of the two kinds of oligonucleotide probes to form a complex including the assist probe and the target analyte that is specifically bonded to the capture substance on the microsphere; and
a step of forming a self assembly substance bonded to the assist probe by using the plural kinds of oligonucleotide probes to form the self assembly substance-bonded particle.

8. The method according to any one of Claims 5 to 7, wherein at least one of the two kinds of oligonucleotide probes is labeled with a fluorescent substance or a substance capable of bonding to a fluorescent substance, and the fluorescent substance and the fluorescent substance on the surface of the microsphere have different fluorescence wavelengths or intensities.

9. The method according to any one of Claims 5 to 8, wherein, in the step (b), the self assembly substance-bonded microsphere is analyzed by flow cytometry.

10. The method according to any one of Claims 5 to 9, wherein the target analyte is a nucleic acid.

11. A kit for detecting the presence of at least one target analyte in a sample, comprising the following (a) and (b):
(a) a microsphere having a fluorescent substance on the surface; and
(b) a pair of oligonucleotide probes including: a first probe that includes three or more of nucleic acid regions including at least a nucleic acid region X, a nucleic acid region Y, and a nucleic acid region Z in the stated order from a 5' end of the first probe; and a second probe that includes three or more of nucleic acid regions including at least a nucleic acid region X' complementary to the nucleic acid region X, a nucleic acid region Y' complementary to the nucleic acid region Y, and a nucleic acid region Z' complementary to the nucleic acid region Z in the stated order from a 5' end of the second probe.

12. The kit according to Claim 11, wherein the microsphere has a capture substance capable of specifically bonding to the target analyte.

13. The kit according to Claim 11 or 12, further comprising an assist probe capable of specifically bonding to the target analyte and having the same base sequence as a part or the whole of a base sequence of one oligonucleotide probe of the pair of oligonucleotide probes.

## Patentansprüche

1. Verfahren zum Herstellen eines Partikels, der an eine Selbstorganisationssubstanz ("selfassemby substance") gebunden ist, umfassend:
a) Bilden einer Selbstorganisationssubstanz, die eine regulären doppelsträngige Konformation aufweist, auf einer Mikrosphäre, die eine fluoreszierenden Substanz auf einer Oberfläche davon aufweist, unter Verwendung von zwei Arten von Oligonukleotidsonden mit komplementären Basensequenzregionen, die miteinander hybridisieren können; und
b) Binden der Selbstorganisationssubstanz auf der Oberfläche der Mikrosphäre zum Herstellen eines Partikels, der an eine Selbstorganisationssubstanz gebunden ist,
wobei die zwei Arten von Oligonukleotidsonden ein Paar von Oligonukleotidsonden sind, umfassend:
(i) einer ersten Sonde, die drei oder mehr Nukleinsäureregionen einschließt, einschließlich mindestens einer Nukleinsäureregion X, einer Nukleinsäureregion Y und einer Nukleinsäureregion Z in der angegebenen Reihenfolge vom 5'-Ende der ersten Sonde; und
(ii) einer zweiten Sonde, die drei oder mehr Nukleinsäureregionen einschließt, einschließlich mindestens einer Nukleinsäureregion X', die komplementär zu der Nukleinsäureregion X ist, einer Nukleinsäureregion Y', die komplementär zu der Nukleinsäureregion Y ist und einer Nukleinsäureregion Z', die komplementär zu der Nukleinsäureregion Z ist, in der angegebenen Reihenfolge vom 5'-Ende der zweiten Sonde.

2. Verfahren nach Anspruch 1, wobei mindestens eine der zwei Arten von Oligonukleotidsonden mit einer fluoreszierenden Substanz oder einer Substanz, die an eine fluoreszierende Substanz binden kann, markiert ist, und die fluoreszierende Substanz und die fluoreszierende Substanz auf der Oberfläche der Mikrosphäre unterschiedliche Fluoreszenzwellenlängen oder -intensitäten haben.

3. Verfahren nach Anspruch 1 oder 2, wobei die Mikrosphäre an einen Zielanalyten gebunden ist, um die Selbstorganisationssubstanz über den Zielanalyten zu binden.

4. Partikel, der an eine Selbstorganisationssubstanz gebunden ist, hergestellt durch das Verfahren nach einem der Ansprüche 1 bis 3.

5. Verfahren zum Nachweisen des Vorhandenseins mindestens eines Zielanalyten in einer Probe, umfassend die folgenden Schritte (a) und (b):
(a) einen ersten Schritt des Bindens einer Mikrosphäre, die eine fluoreszierende Substanz auf ihrer Oberfläche und einen Zielanalyten aufweist, an eine Selbstorganisationssubstanz, die gebildet wurde mit zwei Arten von Oligonukleotidsonden mit komplementären Basensequenzregionen, die über den Zielanalyten miteinander hybridisieren können, um einen Partikel zu bilden, der an eine Selbstorganisationssubstanz gebunden ist; and
(b) einen zweiten Schritt des Analysierens des Partikels, der an eine Selbstorganisationssubstanz gebunden ist, um den Zielanalyten nachzuweisen,
wobei die zwei Arten von Oligonukleotidsonden ein Paar von Oligonukleotidsonden sind, umfassend:
(i) einer ersten Sonde, die drei oder mehr Nukleinsäureregionen einschließt, einschließlich mindestens einer Nukleinsäureregion X, einer Nukleinsäureregion Y und einer Nukleinsäureregion Z in der angegebenen Reihenfolge vom 5'-Ende der ersten Sonde; und
(ii) einer zweiten Sonde, die drei oder mehr Nukleinsäureregionen einschließt, einschließlich mindestens einer Nukleinsäureregion X', die komplementär zu der Nukleinsäureregion X ist, einer Nukleinsäureregion Y', die komplementär zu der Nukleinsäureregion Y ist und einer Nukleinsäureregion Z', die komplementär zu der Nukleinsäureregion Z ist, in der angegebenen Reihenfolge vom 5'-Ende der zweiten Sonde.

6. Verfahren nach Anspruch 5, wobei die Mikrosphäre eine Einfangsubstanz aufweist, die in der Lage ist spezifisch an den Zielanalyten zu binden.

7. Verfahren nach Anspruch 6, wobei der Schritt (a) einschließt:
(i) den Schritt des Verwendens einer Hilfssonde, die in der Lage ist spezifisch an den Zielanalyten zu binden und die die gleiche Basensequenz aufweist wie ein Teil oder die gesamte Basensequenz einer Oligonukleotidsonde der beiden Arten von Oligonukleotidsonden, um einen Komplex zu bilden, der die Hilfssonde und den Zielanalyten einschließt, der spezifisch an die Einfangsubstanz auf der Mikrosphäre bindet; und
(ii) einen Schritt des Bildens einer Selbstorganisationssubstanz, die an die Hilfssonde gebunden ist durch Verwendung einer Vielzahl von Arten von Oligonukleotidsonden, um einen Partikel zu bilden, der an eine Selbstorganisationssubstanz gebunden ist.

8. Verfahren nach einem der Ansprüche 5 bis 7, wobei mindestens eine der beiden Arten von Oligonukleotidsonden mit einer fluoreszierenden Substanz oder einer Substanz, die an eine fluoreszierende Substanz binden kann, markiert ist, und die fluoreszierende Substanz und die fluoreszierende Substanz auf der Oberfläche der Mikrosphäre unterschiedliche Fluoreszenzwellenlängen oder -intensitäten haben.

9. Verfahren nach einem der Ansprüche 5 bis 8, wobei in dem Schritt (b) die Mikrosphäre, die an die Selbstorganisationssubstanz gebunden ist, mittels Durchflusszytometrie analysiert wird.

10. Verfahren nach einem der Ansprüche 5 bis 9, wobei der Zielanalyt eine Nukleinsäure ist.

11. Kit zum Nachweisen des Vorhandenseins mindestens eines Zielanalyten in einer Probe, umfassend die folgenden (a) und (b):
(a) eine Mikrosphäre mit einer fluoreszierenden Substanz auf ihrer Oberfläche und
(b) ein Paar von Oligonukleotidsonden, einschließlich einer ersten Sonde, die drei oder mehr Nukleinsäureregionen einschließt, einschließlich mindestens einer Nukleinsäureregion X, einer Nukleinsäureregion Y und einer Nukleinsäureregion Z in der angegebenen Reihenfolge vom 5'-Ende der ersten Sonde; und einer zweiten Sonde, die drei oder mehr Nukleinsäureregionen einschließt, einschließlich mindestens einer Nukleinsäureregion X', die komplementär zu der Nukleinsäureregion X ist, einer Nukleinsäureregion Y', die komplementär zu der Nukleinsäureregion Y ist und einer Nukleinsäureregion Z', die komplementär zu der Nukleinsäureregion Z ist, in der angegebenen Reihenfolge vom 5'-Ende der zweiten Sonde.

12. Kit nach Anspruch 11, wobei die Mikrosphäre eine Einfangsubstanz aufweist, die in der Lage ist den Zielanalyten spezifisch zu binden.

13. Kit nach Anspruch 11 oder 12, der weiterhin eine Hilfssonde umfasst, die in der Lage ist an den Zielanalyten spezifisch zu binden und die die gleiche Basensequenz aufweist, wie ein Teil oder die gesamte Basensequenz einer Oligonukleotidsonde des Oligonukleotidsondenpaares.

## Revendications

1. Procédé de fabrication d'une particule liée à une substance d'auto-assemblage, comprenant :
la formation d'une substance d'auto-assemblage ayant une conformation de double brin régulière sur une microsphère ayant une substance fluorescente sur une surface de celle-ci par utilisation de deux types de sondes oligonucléotidiques ayant des régions de séquence de bases complémentaires qui peuvent s'hybrider entre elles ; et
lier la substance d'auto-assemblage à la surface de la microsphère pour fabriquer une particule liée à une substance d'auto-assemblage,
dans lequel les deux types de sondes oligonucléotidiques sont constitués d'une paire de sondes oligonucléotidiques comprenant :
une première sonde qui comprend trois ou plus de trois régions d'acide nucléique comprenant au moins une région d'acide nucléique X, une région d'acide nucléique Y, et une région d'acide nucléique Z, dans l'ordre indiqué en partant de l'extrémité 5' de la première sonde ; et
une deuxième sonde qui comprend trois ou plus de trois régions d'acide nucléique comprenant au moins une région d'acide nucléique X' complémentaire de la région d'acide nucléique X, une région d'acide nucléique Y' complémentaire de la région d'acide nucléique Y, et une région d'acide nucléique Z' complémentaire de la région d'acide nucléique Z, dans l'ordre indiqué en partant de l'extrémité 5' de la deuxième sonde.

2. Procédé selon la revendication 1, dans lequel au moins l'un des deux types de sondes oligonucléotidiques est marqué avec une substance fluorescente ou une substance capable de se lier à une substance fluorescente, et la substance fluorescente et la substance fluorescente sur la surface de la microsphère ont des longueurs d'ondes ou des intensités de fluorescence différentes.

3. Procédé selon la revendication 1 ou 2, dans lequel la microsphère est liée à un analyte cible pour que la microsphère soit liée à la substance d'auto-assemblage via l'analyte cible.

4. Particule liée à une substance d'auto-assemblage produite par le procédé de l'une quelconque des revendications 1 à 3.

5. Procédé pour détecter la présence d'au moins un analyte cible dans un échantillon, comprenant les étapes (a) et (b) suivantes :
(a) une première étape consistant à lier une microsphère ayant une substance fluorescente sur la surface et un analyte cible à une substance d'auto-assemblage formée par utilisation de deux types de sondes oligonucléotidiques ayant des régions de séquence de bases complémentaires qui peuvent s'hybrider entre elles via l'analyte cible pour former une particule liée à une substance d'auto-assemblage ; et
(b) une deuxième étape consistant à analyser la particule liée à une substance d'auto-assemblage pour détecter l'analyte cible,
dans lequel les deux types de sondes oligonucléotidiques sont constitués d'une paire de sondes oligonucléotidiques comprenant :
une première sonde qui comprend trois ou plus de trois régions d'acide nucléique comprenant au moins une région d'acide nucléique X, une région d'acide nucléique Y, et une région d'acide nucléique Z, dans l'ordre indiqué en partant de l'extrémité 5' de la première sonde ; et
une deuxième sonde qui comprend trois ou plus de trois régions d'acide nucléique comprenant au moins une région d'acide nucléique X' complémentaire de la région d'acide nucléique X, une région d'acide nucléique Y' complémentaire de la région d'acide nucléique Y, et une région d'acide nucléique Z' complémentaire de la région d'acide nucléique Z, dans l'ordre indiqué en partant de l'extrémité 5' de la deuxième sonde.

6. Procédé selon la revendication 5, dans lequel la microsphère a une substance de capture capable de se lier spécifiquement à l'analyte cible.

7. Procédé selon la revendication 6, dans lequel l'étape (a) comprend :
une étape consistant à utiliser une sonde d'assistance capable de se lier spécifiquement à l'analyte cible et ayant la même séquence de bases qu'une partie ou que la totalité d'une séquence de bases d'une sonde oligonucléotidique des deux types de sondes oligonucléotidiques pour former un complexe comprenant la sonde d'assistance et l'analyte cible qui est spécifiquement lié à la substance de capture sur la microsphère ; et
une étape consistant à former une substance d'auto-assemblage liée à la sonde d'assistance par utilisation des plusieurs types de sondes oligonucléotidiques pour former la particule liée à une substance d'auto-assemblage.

8. Procédé selon l'une quelconque des revendications 5 à 7, dans lequel au moins l'un des deux types de sondes oligonucléotidiques est marqué avec une substance fluorescente ou une substance capable de se lier à une substance fluorescente, et la substance fluorescente et la substance fluorescente sur la surface de la microsphère ont des longueurs d'ondes ou des intensités de fluorescence différentes.

9. Procédé selon l'une quelconque des revendications 5 à 8, dans lequel, dans l'étape (b), la microsphère liée à une substance d'auto-assemblage est analysée par cytométrie de flux.

10. Procédé selon l'une quelconque des revendications 5 à 9, dans lequel l'analyte cible est un acide nucléique.

11. Kit pour détecter la présence d'au moins un analyte cible dans un échantillon, comprenant (a) et (b) suivants :
(a) une microsphère ayant une substance fluorescente sur la surface ; et
(b) une paire de sondes oligonucléotidiques comprenant : une première sonde qui comprend trois ou plus de trois régions d'acide nucléique comprenant au moins une région d'acide nucléique X, une région d'acide nucléique Y, et une région d'acide nucléique Z, dans l'ordre indiqué en partant de l'extrémité 5' de la première sonde ; et une deuxième sonde qui comprend trois ou plus de trois régions d'acide nucléique comprenant au moins une région d'acide nucléique X' complémentaire de la région d'acide nucléique X, une région d'acide nucléique Y' complémentaire de la région d'acide nucléique Y et une région d'acide nucléique Z' complémentaire de la région d'acide nucléique Z, dans l'ordre indiqué en partant de l'extrémité 5' de la deuxième sonde.

12. Kit selon la revendication 11, dans lequel la microsphère a une substance de capture capable de se lier spécifiquement à l'analyte cible.

13. Kit selon la revendication 11 ou 12, comprenant en outre une sonde d'assistance capable de se lier spécifiquement à l'analyte cible et ayant la même séquence de bases qu'une partie ou que la totalité d'une séquence de bases d'une sonde oligonucléotidique de la paire de sondes oligonucléotidiques.
